Europäisches Patentamt

European Patent Office

Office européen des brevets

(11) Publication number: **0 455 560 A2**

(19)

# (12) EUROPEAN PATENT APPLICATION

(21) Application number: 91401178.8

(51) Int. Cl.⁵: **A61K 31/70**

(22) Date of filing: 06.05.91

(30) Priority: 04.05.90 IT 4158990

(43) Date of publication of application:
06.11.91 Bulletin 91/45

(84) Designated Contracting States:
AT BE CH DE DK ES FR GB GR IT LI LU NL SE

(71) Applicant: FIDIA S.p.A.
Via Ponte della Fabbrica 3-A
I-35031 Abano Terme (Padova) (IT)

(72) Inventor: Della Valle, Francesco
Via Cerato 14
I-35100 Padova (IT)
Inventor: Callegaro, Lanfranco
Via Bravi 35
I-35020 Ponte di Brenta Padova (IT)
Inventor: Romeo, Aurelio
Viale Ippocrate 93
I-00161 Rome (IT)

(74) Representative: Hirsch, Marc-Roger
Cabinet Hirsch 34 rue de Bassano
F-75008 Paris (FR)

(54) Use of N-dichloroacetyl lysogangliosides for the selective modulation of the expression and function of CD4 determinants on the cell surface.

(57) Use of N-dichloroacetyl lysogangliosides, such as N-dichloroacetyl lyso $GM_1$, in the manufacture or preparation of a pharmaceutical formulation for selectively modulating the $CD_4$ determinant present on the surface of human cells. This modulation can be used therapeutically to treat neuroim-munopathological conditions or neuroimmunopathology associated with infection by viruses such as the HIV-I virus.

EP 0 455 560 A2

## DETAILED DESCRIPTION

The present invention is directed to a process for inhibiting the immune system mechanisms by selectively modulating the expression of the $CD_4$ determinant on the cell surfaces of human cells by the use of modified gangliosides which cause the $CD_4$ determinants to rapidly and selectively disappear from the cell surface in the presence of serum. By selectively modulating the expression and function of the $CD_4$ determinant on the cell surface, using modified gangliosides which cause the rapid and selective disappearance of $CD_4$, it is possible to eliminate the point of attack and entry of viruses into T-helper cells which leads to the infection of human cells through cell surface $CD_4$ determinants, thus inhibiting the receptoral ability of these cell lines, without inhibiting the other determinants present on the cell surface.

## BACKGROUND OF THE INVENTION

The present invention concerns a process by which the expression and function of a determinant present on the cell surface of human cells is selectively modulated. By the selective modulation of the expression and function of specific determinants, human cells can be protected from debilitating agents, be they of exogenous or endogenous origin, while leaving their function unaltered. The properly-functioning immune system protects the organism against infective agents including viruses, bacteria, parasites and similar agents. The ability to combat such agents or other substances, called antigens and recognized as foreign particles by the immune system, is vital to health and survival. Under normal conditions the immune system is capable of fighting many types of infection and restoring total recovery, but when it is compromised infection can strike unhindered. Malfunction of the immune system can also be manifested by its excessive biological activity, perhaps expressed as an overproduction of its natural components and by its recognizing as antigens even those biological components that are normally present in the organism.

Macrophages and lymphocytes are mainly involved in the immune processes. Macrophages attack and destroy the infective agent while supplying information on its structure to the lymphocytes. There are two categories of lymphocytes. The first, known as B cells, produces antibodies in response to infective agents. Antibodies have specific combination sites which discriminate between the different structures of the single antigens. When an antibody binds to an antigen, their interaction can block the effect of a foreign substance. The second kind of lymphocyte, T cells, produces only membrane receptors, which recognize the antigen structures from information received from the macrophages. When the T cells interact with the antigen, a large number of hormones (lymphokines) are released which can induce inflammation, amplify or suppress the formation of antibodies by the B cells or directly destroy any foreign cells such as tumors or transplanted organs.

The balance and exchange between T cells, B cells and macrophages determine the force of immune response to infection in the individual. It is generally thought that the activities of the immune cells are largely controlled by lymphokines and monokines, each of which has its own distinct chemical properties and functions. The paper "The $CD_4$ Molecule: Function and Structure" by Dalgleish, published in Immunology Today, Volume 7, page 142, 1986, reports that one specific determinant present on the cell surface, for example the $CD_4$ molecule, defines a subpopulation of mature T lymphocytes which, after antigen recognition (restricted to the antigens of the major histocompatibility complex (MHC), class II), leads to a wide variety of regulatory and effector functions. Modification of the $CD_4$ determinant present on the cell surface apparently also causes a negative signal to the T cells and can act as a blocker of T cell function. The antigen $CD_4$ (T4) is a fundamental receptor of the helper T lymphocytes and has been found to play a fundamental role as receptor for the retrovirus associated with AIDS (Dalgleish et al., Nature, 312:763, 1984).

The precise function of the $CD_4$ molecule is not yet altogether clear, but experimental evidence indicates that it may interact with the molecule of the major histocompatibility complex II (MHC-II), to more efficiently mediate recognition by the helper T cells of the positioning targets of the antigen (Gay et al., Nature, 328:626, 1987; Doyle et al., Nature, 330:256, 1988). Moreover, studies have been carried out on the role of the $CD_4$ molecule as a signal transductor in the membrane (Bank et al., J. Exp. Med. 162:1294, 1985). Exposure of the helper T cell to phorbol esters causes internalization of the $CD_4$ molecule with phosphorylation of the cytoplasmic serine residue by the protein kinase C (Hoxie et al., J. Immunol. 137:1194, 1986; Acies et al., J. Biol. Chem. 261:1610, 1986; Hoxie et al., J. Immunol. 140:786, 1986).

A modulating or masking action has also been defined by incubating cells which expressed $CD_4$ with acid glycosphingolipids, such as gangliosides (Offner et al., J. Immunol. 139:3295, 1987; Offner et al., Biochem. Biophys. Res. Commun. 158:1050, 1989; Life Science 1989). It is also possible to obtain this action with gangliosides that have been chemically modified in a suitable way on the functional carboxy group of the sialic acid moiety (Grassi et al., J. Immunol. 1990, in press) and which, in the case of the monosialoganglioside $GM_1$ (Svennerholm's nomenclature, 1963), has been shown to slow down infection by the human immunodeficiency virus HIV-1 when suitable cell lines were used (Chieco Bianchi et al., AIDS 3:501, 1989). This action of modulating or masking $CD_4$ by some gangliosides, such as $GM_1$, $GD_{1a}$, $GD_{1b}$, $GT_{1b}$ or their mixtures or suitable, chemically modified derivatives, has not yet been clarified.

The property which all of these molecules have in common is that their modulating or masking activity on the $CD_4$ receptor disappears completely in in vitro experiments when these are carried out in the presence of serum or its derivatives, even at very low concentrations. This totally excludes the use of these molecules in vivo for the modulation of $CD_4$.

Molecules which selectively modulate or mask $CD_4$ would represent a great asset and benefit in the art if this action were to be verified in the presence of the normal biological components, such as serum or its components. This objective has been met by the present invention, which is characterized by the discovery of certain compounds that are active on the $CD_4$ protein in the presence of serum.

An important property of the biologically active material in humans is its ability to discriminate, for example, by selectively modulating the expression and function of a given determinant present on the cell surface. Antibodies belong to a class of molecules which selectively discriminate, but they may have certain side effects. Chemical compositions, like drugs, are not usually discriminating and can affect many different types of human cells. Consequently, there is a need for chemical compositions which can discriminate and which have the selective ability to modulate the determinants present in human cells so as to prevent infections or other undesirable effects connected with the immune system.

## SUMMARY OF THE INVENTION

The present invention is directed to a process of reversibly inhibiting a biological action in man, by means of the selective modulation of the expression of a determinant, present on the cell surface and known as $CD_4$ for human cells, by the use of certain chemical agents. These chemical agents comprise a composition of modified gangliosides or mixtures of modified gangliosides and natural gangliosides which induce the rapid and selective disappearance of the $CD_4$ molecule on human cell lines. After treatment with these modified gangliosides or mixtures, human cells stop expressing $CD_4$ after a period of time and their biological action is slowed down, for example, its interaction with the HIV virus.

For the purposes of the present invention, the term modified ganglioside as used herein is meant to define a compound obtained by a series of definite and reproducible chemical reactions, starting from single natural gangliosides or mixtures of the same. Selective modulation on human cells is obtained in the presence of serum, which does not neutralize the action of the modified ganglioside.

The modified gangliosides of the present invention are semisynthetic ganglioside analogues and differ from natural gangliosides in that they possess a single N-acyl group in the sphingosine part, namely an N-dichloroacetyl group. These complexes, called N-dichloroacetyl lysogangliosides have already been described in the literature, for example, European Patent Publication No. 0373039 dated June 13, 1990, the disclosure of which is expressly incorporated herein. The preferred N-dichloroacetyl lysoganglioside is N-dichloroacetyl lyso $GM_1$. These compounds are known to have an immunosuppressive activity.

Another objective of the invention is to provide pharmaceutical preparations containing as active substances one or more of the aforesaid derivatives or semisynthetic ganglioside analogues, and particularly compositions containing said N-dichloroacetyl lysogangliosides. Such pharmaceutical preparations can be intended for oral, rectal, vaginal, parenteral or local use. They are therefore in solid or semisolid form, for example, pills, tablets, gelatinous capsules, capsules, suppositories, soft gelatin capsules, gels, microspheres, membranes, pessaries and creams. For parenteral use it is possible to employ those forms intended for intramuscular or subcutaneous administration or for infusions or intravenous or intracerebral injection and, therefore, the pharmaceutical compositions can be in the form of solutions of the active compounds and as freeze-dried powders of the active compounds to be mixed with one or more pharmaceutically acceptable excipients or diluents, which are convenient for the aforesaid uses and osmotically compatible with physiological fluids. For local use preparations in the form of sprays, for example nose sprays, or creams or ointments for topical use can be considered.

Such preparations can contain the active principale and possibly also associations with polymers, for example acidic polysaccharides, such as hyaluronic acid or alginic acid, chemically modified and transformed into manufactured products coming under the name of biomaterials. For preferential, but not limitative purposes, ester derivatives (total or partial) of ethyl, benzyl, or dodecyl alcohol, can be mentioned, as obtained from hyaluronic acid (as described by Fidia in US patent Nos. 4,851,521 and 4,965,353) or from alginic acid (as described by Fidia in the E.P.O. publication No.0251905).

The preparations of the invention can be administered to humans or animals. The invention is particularly applicable to mammals such as human beings. Such preparations contain preferably from 0.01% to 10% by weight of active component for solutions, sprays, ointments and creams, and from 1% to 100% and preferably from 5% to 50% by weight of active compound for preparations in solid form. The doses to be administered depend on individual needs, the desired effect and the chosen administration route.

The invention also includes the therapeutic use of the compositions of the invention, as they relate to the modulation of the expression of the $CD_4$ molecule on the cell. Daily dosages to man by injection (subcutaneous, intramuscular or intracerebral) will vary from 5 mg to 25 mg of active substance per kg of body weight.

The following Examples are given merely as illustrative of the present invention and are not to be considered as limiting. Unless otherwise noted, the percentages therein and throughout the application are by weight.

## EXAMPLE 1

The selective inhibition and disappearance of the expression and function of the $CD_4$ determinants on the cell surface by a composition of modified gangliosides or by a specific class of modified ganglioside molecules has been experimentally demonstrated.

Human lymphocytes were incubated at various concentrations of the modified ganglioside N-dichloroacetyl lyso $GM_1$. The cells were stained using monoclonal antibodies (Mab) specific for $CD_4$, $CD_8$, $CD_3$ ($T_4$, $T_8$, $T_3$ Coulter Clone, Coulter Electronics, Hialeah, Florida, USA) and $OKT_4$ (Ortho Diagnostics, Raritan, New Jersey, USA), these monoclonal antibodies being coupled to fluorescein. By measuring the mean fluorescence intensity (MFI) of the labelled cell population at each concentration of N-dichloroacetyl lyso $GM_1$, it was possible to quantify how much N-dichloroacetyl lyso $GM_1$ is necessary to produce 50% inhibition of the fluorescence ($I_{50}$). The $I_{50}$ value for minor lymphocytes was 5 μM. The concentration of the composition of N-dichloroacetyl lyso $GM_1$ at which the fluorescence intensity measurement was 0% was resolved experimentally.

Specifically, human cells were incubated with saline solution at various concentrations of N-dichloroacetyl lyso $GM_1$ in the presence of serum at 37°C for one hour. The cells were then washed, incubated with suitable monoclonal antibodies, fixed with 1% formalin and analyzed by MFI after excitation at 488 mm laser length. Values are reported in Table 1.

TABLE 1

| Concentration of N-dichloroacetyl lyso GM$_1$ ($\mu$g/ml) | Mean fluorescence intensity (% of positive control) | |
| --- | --- | --- |
| 2 | anti CD$_4$ Mab | 100 |
| | anti CD3 Mab | 100 |
| 5 | anti CD$_4$ Mab | 60 |
| | anti CD$_3$ Mab | 100 |
| 10 | anti CD$_4$ Mab | 28 |
| | anti CD$_3$ Mab | 100 |
| 25 | anti CD$_4$ Mab | 0 |
| | anti CD$_3$ Mab | 100 |
| 50 | Mab anti CD$_4$ | 0 |
| | Mab anti CD$_3$ | 100 |

The values reported in Table 1 show that N-dichloroacetyl lyso GM$_1$ selectively modulates the CD$_4$ receptor without altering the CD$_3$ receptor.

EXAMPLE 2

Molt 3 cells (5x10$^6$) were incubated with various concentrations of N-dichloroacetyl lyso GM$_1$ (from 1 μg/ml to 250 μg/ml) for 60 minutes at 37°C in the presence of BMS (BMS Serum Substitute, Biochrom. Kg. Seromed, Berlin, Germany). After incubation the percentage of CD$_4$ expressed was shown by a specific Mab for CD$_4$ (T$_4$ Coulter Clone, Coulter Electronics, Hialeah, Florida, USA). For a positive control, an experiment-was carried out with monosialoganglioside GM$_1$ under the same experimental conditions as reported for N-dichloroacetyl lyso GM$_1$.

Table 2 reports the percentage of CD$_4$ still expressed in the Molt 3 cells after incubation with different chemical molecules at different concentrations, as described in Example 1. The CD$_4$ expressed in the absence of the chemical agents during the experiment was 89%.

## TABLE 2

|  | 1μg/ml | 5μg/ml | 25μg/ml | 125μg/ml |
|---|---|---|---|---|
| N-dichloroacetyl lyso $GM_1$ | 75% | 74% | 47% | 13% |
| $GM_1$ | 88% | 81% | 52% | 0% |

The results reported in Table 2 indicate that the modulating effect of $GM_1$ and its chemical derivative has the function of a dose-response curve.

## EXAMPLE 3

Molt 3 cells ($5 \times 10^6$) were incubated with different concentrations of N-dichloroacetyl lyso $GM_1$ (from 1 μg/ml to 125 μg/ml) for 60 minutes at 37°C in the presence of different concentrations of fetal calf serum (FCS) (from 1% to 5%). After incubation the percentage of $CD_4$ expressed was shown by a specific Mab for $CD_4$ ($T_4$ Coulter Clone, Coulter Electronics, Hialeah, Florida, USA).

As a positive control, an experiment with the monosialoganglioside $GM_1$ was carried out under the same experimental conditions as reported for N-dichloroacetyl lyso $GM_1$.

Table 3 shows the data relative to the percentage of $CD_4$ still expressed in the Molt 3 cells-after incubation with the two molecules at their different concentrations and at different concentrations of fetal calf serum. The $CD_4$ expressed in the absence of the chemical agents during the experiment was 84%.

## TABLE 3

### N-DICHLOROACETYL LYSO $GM_1$

| Concentration | 1μg/ml | 25μg/ml | 75μg/ml | 125μg/ml |
|---|---|---|---|---|
| FCS 1% | 70% | 44% | 47% | 30% |
| 2% | 73% | 55% | 50% | 49% |
| 5% | 77% | 64% | 55% | 50% |

$GM_1$

| Concentration | $1\mu g/ml$ | $25\mu g/ml$ | $75\mu g/ml$ | $125\mu g/ml$ |
|---|---|---|---|---|
| FCS 1% | 82% | 80% | 50% | 10% |
| 2% | 84% | 83% | 80% | 25% |
| 5% | 85% | 81% | 83% | 72% |

The results reported in Table 3 indicate that in the case of the compound N-dichloroacetyl lyso $GM_1$ increased serum concentrations do not alter its modulating action on the $CD_4$ receptor, while in the case of $GM_1$ this modulating action disappears in the presence of serum.

Typical pharmaceutical formulations are shown in the following Examples:

Preparation No. 1 - one 2-ml ampoule contains:
- N-dichloroacetyl lyso GM$_1$     mg   5
- sodium chloride     mg 16
- citrate buffer pH 6 in water for injection to a volume of     ml   2

Preparation No. 2 - 100 g of ointment contain:
- N-dichloroacetyl lyso GM$_1$ (in 5 g of mixed phospholipid liposomes)     g   4.0
- polyethylene glycol monostearate     g   1.5
- glycerin     g   1.5
- ester of p-oxybenzoic acid     mg   125
- water     g 72.9

Preparation No. 3 - Cream containing a total ester of hyaluronic acid with ethyl alcohol, of which 100 gr contain:
- N-dichloroacetyl lyso GM$_1$     g   1.000
- total ester of hyaluronic acid with ethyl alcohol     g   0.200
- polyethylene glycol monostearate 400     g   10.000
- cetiol V     g   5.000
- lanette SX     g   2.000
- paraoxybenzoate of mehtyl     g   0.075
- paraoxybenzoate of propyl     g   0.050
- sodium dihydroacetate     g   0.100
- glycerine F.U.     g   1.500
- sorbitol 70     g   1.500
- test cream     g   0.050
- water for injections to a volume of     g 100.000

Preparation No. 4 - Cream containing a partial ester of hyaluronic acid with ethyl alcohol, (75% of esterified carboxylic groups - 25% of salified carboxylic groups) of which 100 gr contain:
- N-dichloroacetyl lyso GM$_1$     g   1.000
- partial ester of hyaluronic acid with ethyl alcohol     g   0.200
- polyethylene glycol monostearate 400     g   10.000
- cetiol V     g   5.000
- lanette SX     g   2.000
- paraoxybenzoate of methyl     g   0.075
- paraoxybenzoate of propyl     g   0.050
- sodium dihydroacetate     g   0.100
- glycerine F.U.     g   1.500
- sorbitol 70     g   1.500
- test cream     g   0.050
- water for injections to a volume of     g 100.000

Preparation No. 5 - Cream containing a total ester of hyaluronic acid with benzyl alcohol, of which 100 gr contain:
- N-dichloroacetyl lyso GM$_1$ ........................ g 1.000
- total ester of hyaluronic acid
  with benzyl alcohol ........................ g 0.200
- polyethylene glycol monostearate 400 ........ g 10.000
- cetiol V ........................ g 5.000
- lanette SX ........................ g 2.000
- paraoxybenzoate of methyl ........................ g 0.075
- paraoxybenzoate of propyl ........................ g 0.050
- sodium dihydroacetate ........................ g 0.100
- glycerine F.U. ........................ g 1.500
- sorbitol 70 ........................ g 1.500
- test cream ........................ g 0.050
- water for injections to a volume of ........ g 100.000

Preparation No. 6 - Cream containing a partial ester of hyaluronic acid with benzyl alcohol (75% of esterified carboxylic groups - 25% of salified carboxylic groups), of which 100 gr contain:
- N-dichloroacetyl lyso GM$_1$ ........................ g 1.000
- partial ester of hyaluronic acid
  with benzyl alcohol ........................ g 0.200
- polyethylene glycol monostearate 400 ........ g 10.000
- cetiol V ........................ g 5.000
- lanette SX ........................ g 2.000
- paraoxybenzoate of methyl ........................ g 0.075
- paraoxybenzoate of propyl ........................ g 0.050
- sodium dihydroacetate ........................ g 0.100
- glycerine F.U. ........................ g 1.500
- sorbitol 70 ........................ g 1.500
- test cream ........................ g 0.050
- water for injections to a volume of ........ g 100.000

Preparation No. 7 - Cream containing a total ester of alginic acid with ethyl alcohol of which 100 gr contain:
- N-dichloroacetyl lyso GM$_1$ ........................ g 1.000
- total ester of alginic acid
  with ethyl alcohol ........................ g 0.200
- polyethylene glycol monostearate 400 ........ g 10.000
- cetiol V ........................ g 5.000
- lanette SX ........................ g 2.000
- paraoxybenzoate of methyl ........................ g 0.075
- paraoxybenzoate of propyl ........................ g 0.050
- sodium dihydroacetate ........................ g 0.100
- glycerine F.U. ........................ g 1.500
- sorbitol 70 ........................ g 1.500
- test cream ........................ g 0.050
- water for injections to a volume of ........ g 100.000

Preparation No. 8 - Cream containing a partial ester of alginic acid with ethyl alcohol (50% of esterified carboxylic groups - 50% of salified carboxylic groups) of which 100 gr contain:

- N-dichloroacetyl lyso GM$_1$                              g  1.000
- partial ester of alginic acid
  with ethyl alcohol                                        g  0.200
- polyethylene glycol monostearate 400                     g 10.000
- cetiol V                                                  g  5.000
- lanette SX                                                g  2.000
- paraoxybenzoate of methyl                                 g  0.075
- paraoxybenzoate of propyl                                 g  0.050
- sodium dihydroacetate                                     g  0.100
- glycerine F.U.                                            g  1.500
- sorbitol 70                                               g  1.500
- test cream                                                g  0.050
- water for injections to a volume of                      g 100.000

Preparation No. 9 - Cream containing a total ester of alginic acid with benzyl alcohol of which 100 gr contain:

- N-dichloroacetyl lyso GM$_1$                              g  1.000
- total ester of alginic acid
  with benzyl alcohol                                       g  0.200
- polyethylene glycol monostearate 400                     g 10.000
- cetiol V                                                  g  5.000
- lanette SX                                                g  2.000
- paraoxybenzoate of methyl                                 g  0.075
- paraoxybenzoate of propyl                                 g  0.050
- sodium dihydroacetate                                     g  0.100
- glycerine F.U.                                            g  1.500
- sorbitol 70                                               g  1.500
- test cream                                                g  0.050
- water for injections to a volume of                      g 100.000

Preparation No. 10 - Cream containing a partial ester of alginic acid with benzyl alcohol (50% of esterified carboxylic groups - 50% of salified carboxylic groups) of which 100 gr contain:

- N-dichloroacetyl lyso GM$_1$                              g  1.000
- partial ester of alginic acid
  with benzyl alcohol                                       g  0.200
- polyethylene glycol monostearate 400                     g 10.000
- cetiol V                                                  g  5.000
- lanette SX                                                g  2.000
- paraoxybenzoate of methyl                                 g  0.075
- paraoxybenzoate of propyl                                 g  0.050
- sodium dihydroacetate                                     g  0.100
- glycerine F.U.                                            g  1.500
- sorbitol 70                                               g  1.500
- test cream                                                g  0.050
- water for injections to a volume of                      g 100.000

## Claims

1. Use of N-dichloroacetyl lysoganglioside compounds in the manufacture or preparation of a pharmaceutical formulation for modulating the $CD_4$ determinants present on the surface of human cells in vivo.

2. Use of N-dichloroacetyl lysoganglioside compounds according to claim 1, wherein the ganglioside compound is N-dichloroacetyl lyso $GM_1$.

3. Use of N-dichloroacetyl lysoganglioside compounds according to claim 1 or claim 2, wherein the human cells include brain glia cells, human T-helper cells, human epithelial cells or human macrophages.

4. Use of N-dichloroacetyl lysoganglioside compounds according to claim 1 or claim 2, wherein the modulation is selective for $CD_4$ determinants without affecting other determinants present on the cell surface.

5. Use of N-dichloroacetyl lysoganglioside compounds according to claim 1 or claim 2 for selectively modulating the expression of the $CD_4$ determinant present on the surface of human cells, wherein the modulation is maintained by additional doses of N-dichloroacetyl lysoganglioside compound to prevent re-expression of $CD_4$ and wherein removal of the N-dichloroacetyl lysoganglioside compound leads to the re-expression of $CD_4$.

6. Use of N-dichloroacetyl lysoganglioside compounds according to claim 1 or claim 2 for the treatment of neuroimmunopathological conditions.

7. Use of N-dichloroacetyl lysoganglioside compounds according to claim 6 wherein the neuroimmunopathology is multiple sclerosis.

8. Use of N-dichloroacetyl lysoganglioside compounds according to claim 6 wherein the neuroimmunopathology is polyradiculoneuropathy.

9. Use of N-dichloroacetyl lysoganglioside compounds according to claim 6 wherein the neuroimmunopathology is associated with infection by infectious viruses.

10. Use of N-dichloroacetyl lysoganglioside compounds according to claim 9 wherein the virus is the HIV-I virus.

11. Use of N-dichloroacetyl lysoganglioside compounds according to claim 6 wherein the neuroimmunopathology is a chronic neurodegenerative disease or a chronic immunological disease affecting the nervous system.

12. The use according to any of claims 3-11 wherein the ganglioside compound is N-dichloroacetyl lyso $GM_1$.